# EUROPEAN PATENT APPLICATION

(11) **EP 4 524 138 A1**
(43) Date of publication of application: **19.03.2025**
(21) Application number: 23802830.2
(22) Date of filing: 08.05.2023
(51) Int. Cl.: C07D 407/12

(54) **METHOD FOR PREPARING GLUCOPYRANOSYL-CONTAINING COMPOUND**

(30) Priority: 12.05.2022 CN 202210515612; 12.05.2022 CN 202210515735
(71) Applicant: Zhejiang Huahai Pharmaceutical Co., Ltd., Taizhou, Zhejiang 317024 (CN)
(72) Inventor: ZHENG, Chenguang, Taizhou, Zhejiang 317024 (CN); WANG, Tao, Taizhou, Zhejiang 317024 (CN); SHI, Hu, Taizhou, Zhejiang 317024 (CN); JIN, Yehua, Taizhou, Zhejiang 317024 (CN); ZHU, Wei, Taizhou, Zhejiang 317024 (CN); MENG, Yanhua, Taizhou, Zhejiang 317024 (CN)
(74) Representative: Botti & Ferrari S.p.A.
(86) International application number: PCT/CN2023/092648
(87) International publication number: WO 2023/217058

(57) **Abstract**

The present invention relates to a method for preparing a compound as shown in a formula II, comprising: in an organic solvent, reacting a compound shown in formula III with a compound shown in formula IV under the action of organic metal lithium; performing water quenching to obtain a mixed solution, layering same, separating out an organic phase, concentrating, and reacting same with methanol under an acidic condition so as to obtain formula II. The method provided by the present invention requires a mild quenching condition, is low in acid consumption, and the produced product is high in purity.

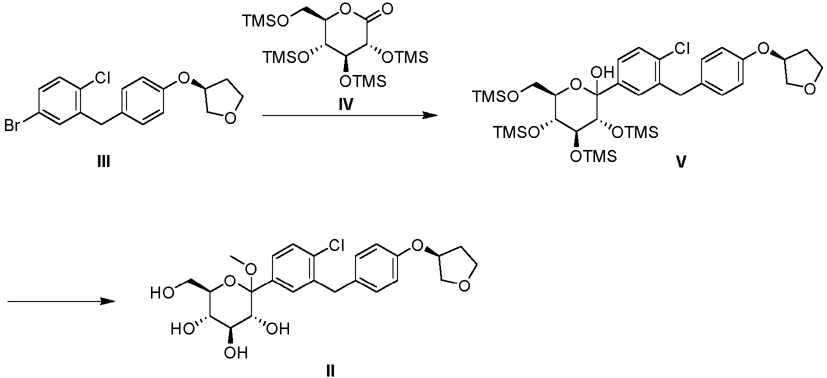

## Description

This application claims priority to Chinese Patent Application No. 202210515612.9, filed with China National Intellectual Property Administration on May 12, 2022 and entitled "METHOD FOR PURIFYING GLUCOPYRANOSYL-CONTAINING COMPOUND", and Chinese Patent Application No. 202210515735.2, filed with China National Intellectual Property Administration on May 12, 2022 and entitled "METHOD FOR PREPARING GLUCOPYRANOSYL-CONTAINING COMPOUND", the whole content of which is incorporated herein by reference.

### Technical Field

The present invention relates to the field of chemical pharmaceutical manufacturing, and more particularly to a method for preparing a glucopyranosyl-containing compound.

### Background Art

The compound of 1-chloro-4-(β-D-glucopyranos-1-yl)-2-[4-((S)-tetrahydrofuran-3-yloxy)-benzyl]-benzene (hereinafter referred to as "compound I") is disclosed in International Patent Application WO 2005/092877 and has a chemical structure of formula I:

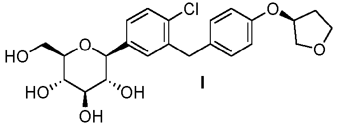

The compound described here has a valuable inhibitory effect on sodium-dependent glucose cotransporters (SGLTs), especially for SGLT2.

International Patent Application WO 2006/120208 discloses a method for preparing compound I, comprising reacting a compound of formula II with a reducing reagent in the presence of a Lewis acid to obtain the compound of formula I. The structural formula of formula II is as shown below:

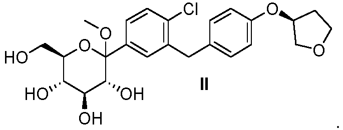

In Example XVII therein, a method for preparing the compound II is disclosed, comprising an addition reaction of a compound represented by formula III with a compound represented by formula IV under the action of tert-butyllithium, and then adding methanol and a methanesulfonate solution for reaction, so as to obtain the compound represented by formula II. The synthetic route is as shown below:

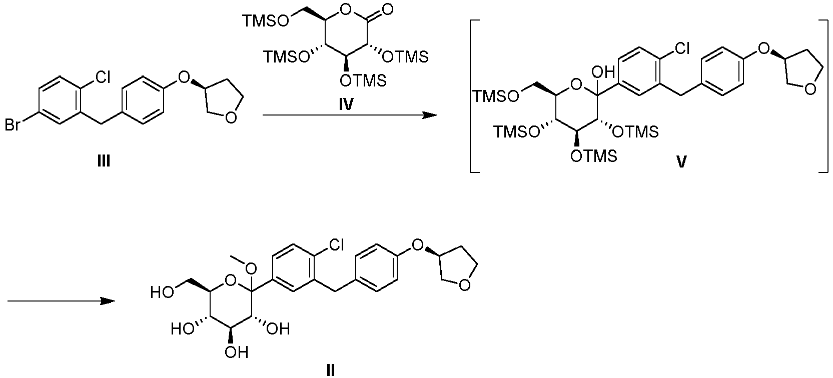

In the process described above, the reaction is quenched directly using MeSO₃H/MeOH, and deprotection and methyl etherification are carried out simultaneously. The intense heat release during the quenching process makes it difficult to control temperature. Additionally, a large amount of methanesulfonic acid is used, the reaction is time-consuming, the conversion rate is low, and a large amount of the intermediate products is retained.

For the synthesis of compound I, for example, according to WO 2006/120208, some impurities present in the final substance are observed. Furthermore, it has been found that although the crystallisation method, as disclosed in WO 2011/039107, reduces the content of impurities and increases the purity of the compound, the results obtained are still unsatisfactory.

### Summary of the Invention

A first aspect of the present invention provides a method for preparing a compound represented by formula II, 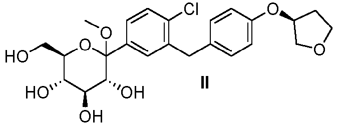 comprising the following steps:
(a) in an organic solvent, reacting a compound represented by formula III 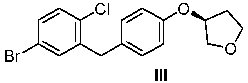 with a compound represented by formula IV under the action of organic metal lithium 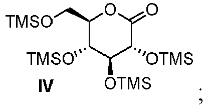
(b) quenching the reaction of step (a) with water to obtain a mixed solution;
(c) subjecting the mixed solution obtained in step (b) to phase separation into an organic phase and an aqueous phase, and concentrating the organic phase to obtain a compound represented by formula V: 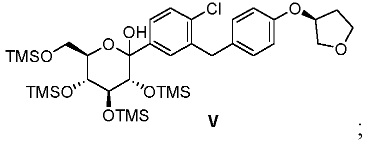 and
(d) reacting the compound represented by formula V obtained in step (c) with methanol under an acidic condition to obtain the compound represented by formula II.

In some embodiments, the organic metal lithium in step (a) is selected from n-butyllithium, tert-butyllithium, or n-hexyllithium.

In some embodiments, the acid in step (d) is selected from methanesulfonic acid, toluenesulfonic acid, sulfuric acid, acetic acid, trifluoroacetic acid, and hydrochloric acid.

In some embodiments, the organic solvent in step (a) is selected from toluene, tetrahydrofuran, methyltetrahydrofuran, hexane, heptane, dioxane, dimethyl sulfoxide, dichloromethane, or any mixture thereof.

In some embodiments, step (b) is carried out in such a manner that the reaction solution of step a) is slowly added to water that was pre-cooled to 0-5°C, with the temperature of quenching being controlled at 0-10°C.

In some embodiments, the phase separation in step (c) is carried out in such a manner that the mixed solution obtained in step (b) is heated to 20-30°C with stirring, so as to separate the organic phase and the aqueous phase.

In some embodiments, the addition reaction in step (a) is carried out at a temperature of -90°C to -60°C, preferably -80°C to -70°C; and the methylation in step (d) is carried out at a temperature of -80°C to 40°C.

The main advantages of the first aspect of the present invention are as follows: different from the operation modes in the prior art, in the present invention, it is unnecessary to add acid when quenching the reaction, and the quenching condition is mild, making it easy to control temperature; the separation of the organic phase from the aqueous phase and the removal of excessive alkali and inorganic impurities can be achieved successfully just by heating the reaction solution; for the deprotection and methyl etherification reactions, the amount of acid used is low, and the reaction time is short; and the methyl-etherified product has high purity, with relatively few residual intermediate-state hemiketal impurity (formula VI) and five-membered ring isomer (formula VII) in the product. The structure of the hemiketal impurity (formula VI) and the five-membered ring isomer impurity (formula VII) in the present invention is as follows:

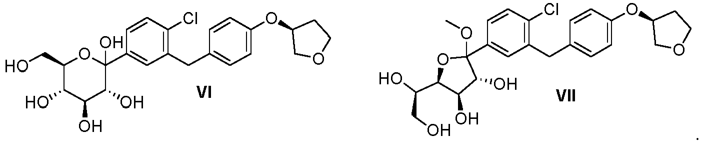

The present invention further provides a method for preparing a compound represented by formula I, which comprises steps (a) - (d) described above, and further comprises a step of:
(e) reacting the compound II with a reducing reagent in the presence of a Lewis acid in a solvent to obtain the compound represented by formula I:

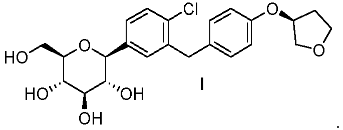

The method for preparing I described above can also be carried out with reference to prior art, for example, the method described in WO 2006/120208.

A further aspect of the present invention provides a method for effectively purifying the compound I, especially a method for purifying the compound with lower costs and higher yield in commercial applications.

The method for purifying the compound represented by formula I provided by the present invention comprises the following steps:
(f) adding the crude product of the compound represented by formula I to a mixed solvent of an organic solvent and water, and heating same with stirring to form a mixed solution;
(g) optionally, gradually cooling the mixed solution obtained in step (f) to crystallise the compound; and
(h) filtering same to obtain the crystals of the compound represented by formula I; wherein
   the organic solvent is selected from ethyl acetate and dichloromethane.

In some embodiments, the crude product of the compound represented by formula I is obtained by a method comprising steps (a) - (d) described above, and further comprising a step of: (e) reacting the compound II with a reducing reagent in the presence of a Lewis acid in a solvent to obtain the compound represented by formula I, and removing the solvent to obtain the crude product of the compound of formula I.

In some embodiments, the volume-to-weight ratio of ethyl acetate to the compound represented by formula I is 1.0 mL/g - 3.0 mL/g, preferably 1.5 mL/g - 2.0 ml/L.

In some embodiments, the volume-to-weight ratio of dichloromethane to the compound represented by formula I is 1.5 mL/g - 4.0 ml/L, preferably 2.0 mL/g - 3.0 ml/L.

In some embodiments, the weight ratio of ethyl acetate to water is in a range of 1 : 10 to 5 : 1, preferably in a range of 1 : 8 to 1 : 1, further preferably in a range of 1 : 6 to 1 : 2, such as 1 : 6, 1 : 5, 1 : 4, 1 : 3, 1 : 2 or any range between them, still further preferably in a range of 1 : 2 to 1 : 3.3.

In some embodiments, the weight ratio of dichloromethane to water is in a range of 1 : 10 to 10 : 1, preferably in a range of 1 : 5 to 5 : 1, further preferably in a range of 1 : 3 to 4 : 1, such as 1 : 3, 1 : 2, 1 : 1, 2 : 1, 3 : 1, 4 : 1, or any range between them, still further preferably in a range of 1 : 2 to 1.33 : 1.

In some embodiments, in step (f), when the organic solvent is ethyl acetate, the temperature of heating with stirring is 55-65°C, and when the organic solvent is dichloromethane, the temperature of heating with stirring is 25-45°C.

In step (g), it is preferred to lower the temperature to obtain high yields of the crystals of compound represented by formula I. The temperature may be lowered continuously or via a predetermined cooling gradient.

In some embodiments, the cooling process in step (g) includes lowering the temperature to 0-10°C.

In some embodiments, the cooling method in step (g) includes gradually lowering the temperature to 0-5°C.

In step (g), when dichloromethane is used, cooling is optional.

The duration of step (g) may be about 30 min to 48 h, preferably about 3-6 h.

Step (g) may be carried out with or without stirring.

By using the purification method according to the present invention, a higher yield of target compound I with high purity can be obtained with a lower amount of an organic solvent, very effectively reducing the impurities shown by the formulae VII and IX below in the crystalline compound:

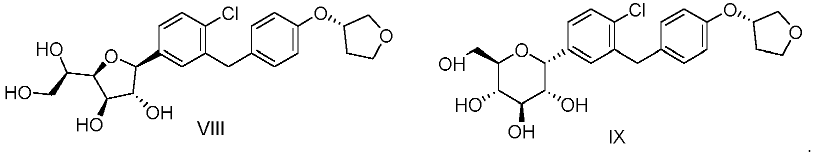

### DETAILED DESCRIPTION OF EMBODIMENTS

The present invention is further described below in conjunction with specific examples.

In the present invention, unless otherwise stated, the scientific and technical terms used herein have the meanings commonly understood by those skilled in the art. Moreover, the laboratory operation procedures used herein are all conventional steps widely used in the corresponding fields.

The terms "have", "comprise", and "include" shall be construed as openended, indicating the presence of the enumerated element but not excluding the presence, occurrence, or addition of any other element or elements not enumerated.

All the ranges recited herein are inclusive of those endpoints of the enumerated ranges between the two values. All the values enumerated herein, whether or not indicated, include the extent of the expected experimental error, technical error, and instrument error of the given technique used to measure the value.

In the present invention, % is a percentage of weight/weight (w/w), if not otherwise stated.

Unless otherwise stated, any numerical value, such as the amount of a solvent or a range of the amount of a solvent described herein, should be understood as being modified by the term "about" in all cases, indicating that such values can vary within a range to some extent. When no range (for example, an error range or a standard deviation of the mean value given in a chart or data table) is provided, the term "about" should be understood as denoting a broader range that includes the stated value, and an included range from which the number can be derived by rounding where significant numbers are taken into account, as well as a range of ±10% of the stated value.

### HPLC method for compound II:

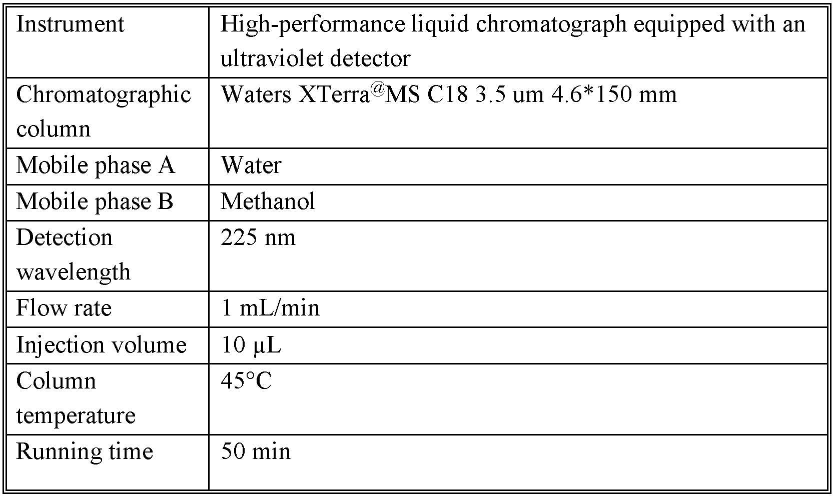

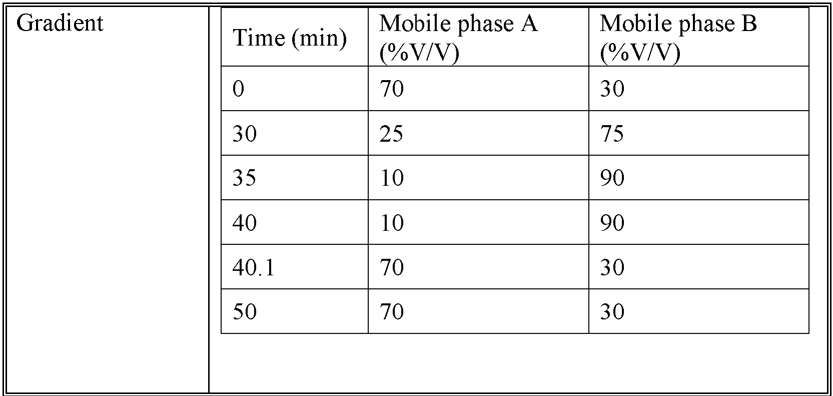

### HPLC method for compound I:

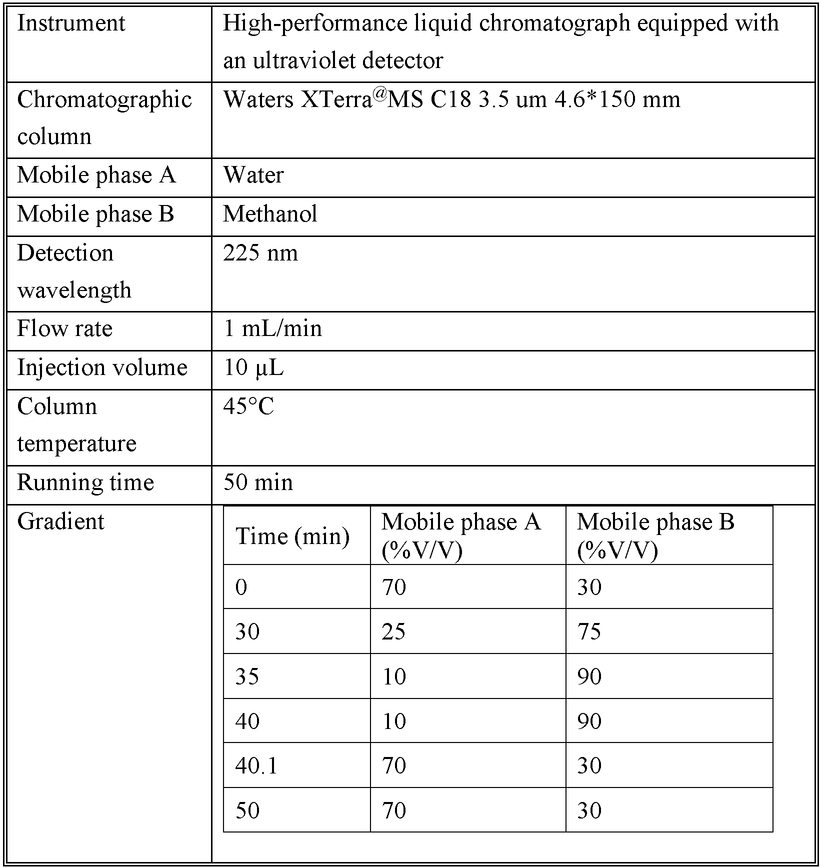

### Example 1

### Preparation of 1-chloro-4-( D-glucopyranos-1-yl)-2-[4-((S)-tetrahydrofuran-3-yloxy)-benzyl]-benzene

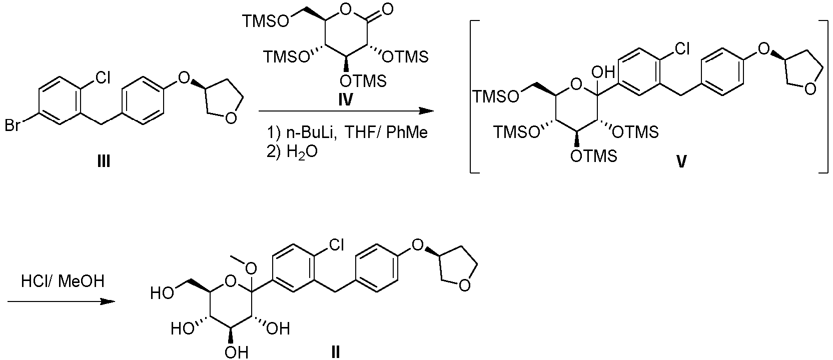

Under the protection of nitrogen, tetrahydrofuran (96 ml) was added to a 1 L four-neck round-bottom flask; a 2.5M solution of n-butyllithium (33 ml, 84.9 mmol) in n-hexane was added dropwise at -78°C; then a solution of compound III (24 g, 65.3 mmol) in toluene (192 ml) was added dropwise, and after completion of the addition, stirring was continued for 30 min; then a solution of compound IV (39.6 g, 84.9 mmol) in toluene (48 ml) was added dropwise, with the addition rate being controlled so that the temperature of the reaction solution was maintained at -70°C or lower; and after completion of the addition, the solution was stirred for 1.5 h, with the temperature being maintained at -80°C to -70°C. The reaction solution was added via a duct to water (120 ml) that was pre-cooled to 0-5°C, with the temperature of quenching being controlled at 0-10°C; and after completion of the addition, the reaction solution was heated to 20-30°C with stirring, and then transferred to a separatory funnel to separate the phases. The organic phase was directly concentrated to obtain a yellow oil (formula V), and the aqueous phase was found to have no compound V detected by TLC. The residue was dissolved in methanol (120 ml), and 11% HCl-methanol (21.6 g, 65.3 mmol) was added dropwise; after completion of the addition, the mixture was stirred at 20-25°C for 3 h; and then the reaction solution was added dropwise to a 5% aqueous sodium bicarbonate solution (120 ml) that was pre-cooled to 0-5°C, with the temperature controlled at 10°C or lower, followed by concentration to remove methanol. Dichloromethane (120 ml) was added to the residue for extraction, followed by phase separation, and the aqueous phase was then extracted with dichloromethane (60 ml) one more time; and then the organic phases were combined and washed by adding water (120 ml) thereto, followed by separation and the concentration of the organic phase to obtain the compound II (31.4 g).

### Comparative examples:

The method of Example 1 was followed, except that water was replaced with a quenching solvent given in Table 1; the resulting compound V was reacted with methanol under an acidic condition to obtain the compound represented by formula II; and the reaction solution was added dropwise to water for quenching, followed by deprotection and methyl etherification to obtain the compound II.

**Table 1 Comparison of results of various quenching solvents and quality of resulting products of formula II**

| Example | Quenchin g solvent | Quenc hing temper ature | System in step d | Amo unt of acid used in step d | Rea ctio n tim e in step d | Purit y | Hem iketa l impu rity | Five-mem bered ring isome r |
|---|---|---|---|---|---|---|---|---|
| Example 1 | Water | 0-10°C | HCl/met hanol | 1.0 eq | 2 h | 91.4 8% | 0.23 % | 1.99 % |
| Compar ative example 1 | 10% aqueous citric acid solution | 0-10°C | HCl/met hanol | 1.0 eq | 4 h | 87.7 0% | 2.67 % | 2.88 % |
| Compar ative example 1 | Methanes ulfonic acid/meth anol | -80°C to - 70°C | Methane sulfonic acid/met hanol | 4.0 eq | 16 h | 79.4 0% | 1.90 % | 3.14 % |
| Compar ative example 2 | Hydrochl oric acid/meth anol | -80°C to - 70°C | Hydroch loric acid/met hanol | 4.0 eq | 5 h | 78.2 1% | 2.33 % | 4.07 % |
| Compar ative example 3 | Trifluoro acetic acid/meth anol | -80°C to - 70°C | Trifluor oacetic acid/met hanol | 4.0 eq | 20 h | 71.2 8% | 2.78 % | 3.65 % |
| Compar ative example 4 | 10% aqueous tartaric acid solution | 0-10°C | HCl/met hanol | 1.0 eq | 4 h | 83.5 1% | 2.01 % | 2.75 % |

As can be seen from Table 1, for the method of Example 1, no reagents are required rather than water, the quenching condition is mild, the separation of the product from the aqueous phase and the removal of excess alkali and inorganic impurities can be achieved just by heating the mixed solution, the amount of acid used in the deprotection and methyl etherification step (step d) is low, the reaction time is short, the purity of the final product is high, and the amounts of main impurities are low.

### Example 2: Preparation method of compound I

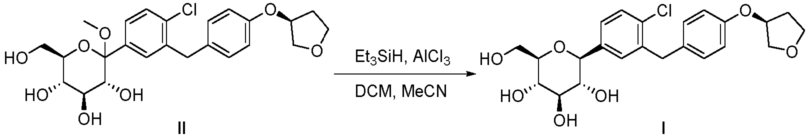

Under the protection of nitrogen, anhydrous aluminum trichloride (14 g, 105 mmol) and dichloromethane (42 mL) were charged to a four-neck flask and cooled to 10°C; and with the temperature controlled at 10-30°C, acetonitrile (60 mL) was added dropwise until the system was clear. Triethylsilane (14.0 g, 120 mmol) was then added dropwise; then with the temperature controlled at 10-20°C, solutions of compound **II** (24.0 g, 50 mmol) in dichloromethane (42 mL) and acetonitrile (60 mL) were added dropwise; and after completion of the addition, the solution was heated to 20-25°C and stirred for 1-2 h. The reaction solution was then cooled to 10°C or lower, and water (120 mL) was added dropwise for quenching. After completion of the addition, the solution was heated to 40-50°C and stirred until dissolved and clear, followed by phase separation. The aqueous phase was extracted with ethyl acetate (48 mL) one more time, and the organic phases were combined and concentrated to obtain the residue of compound I (24.0 g, HPLC purity 85.75%, impurity **VIII:** 4.50%, impurity **IX:** 0.95%) as a yellow solid.

### Example 3: an example of purification using ethyl acetate and water

Water (120 mL) and ethyl acetate (36 mL) were added to the residue obtained in Example 2, and the resulting solution was heated to 55-65°C and stirred until dissolved and clear, and then gradually cooled to 0-5°C and stirred for 2-5 h. The solution was filtered to collect solids, and the filter cake was sequentially rinsed with water (24 mL) and ethyl acetate (6 mL) that was pre-cooled to 0-5°C and then dried in an oven at 45-55°C under vacuum to obtain off-white to pale yellow crystals (17.6 g, 39 mmol), with a yield of 78% (calculated from compound II), an HPLC purity: 99.13%, impurity **VIII:** 0.30%, and impurity **IX:** 0.09%.

### Example 4: an example of purification using dichloromethane and water

Water (64 mL) and dichloromethane (48 mL) were added to the residue obtained in Example 2, and the resulting solution was heated to 35-45°C and stirred for 90 min, and then gradually cooled to 0-5°C and stirred for 2-5 h. The solution was filtered to collect solids, and the filter cake was rinsed with dichloromethane (6 mL) that was pre-cooled to 0-5°C and then dried in an oven at 45-55°C under vacuum to obtain off-white to pale yellow crystals (18.5 g, 41 mmol), with a yield of 82% (calculated from compound II), an HPLC purity: 99.22%, impurity **VIII:** 0.50%, and impurity **IX:** 0.10%.

### Example 5: results of examples of purification using ethyl acetate and water in varying ratios, amounts, etc.:

The purification method of Example 3 was followed, except that the ethyl acetate-water ratios and amounts were varied, so as to refine the crude compound **I** obtained in Example 2. The data on yield (calculated from compound II), purity, and main impurities were summarized, and the results were shown in Table 2.

**Table 2 Results of refining using ethyl acetate and water in varying ratios and amounts**

| Amount of ethyl acetate | Ethyl acetate : Water (weight ratio) | Yield | Purity | Impurity **VIII** | Impurity **IX** |
|---|---|---|---|---|---|
| 3.0 mL/g | 1 : 2 | 69% | 99.10% | 0.27% | 0.08% |
| 2.0 mL/g | 1 : 2 | 76% | 99.15% | 0.29% | 0.08% |
| 1.5 mL/g | 1 : 3.3 | 78% | 99.13% | 0.30% | 0.09% |
| 1.0 mL/g | 1 : 6 | 78% | 98.55% | 0.58% | 0.20% |

### Example 6: Results of examples of purification using dichloromethane and water in varying ratios, amounts, etc.:

The purification method of Example 4 was followed, except that the dichloromethane-water ratios and amounts were varied, so as to refine the crude compound **I** obtained in Example 2. The data on yield (calculated from compound II), purity, and main impurities were summarized, and the results were shown in Table 3.

**Table 3 Results of refining using dichloromethane and water in varying ratios and amounts**

| Amount of dichloromethane | Dichloromethane : Water (weight ratio) | Yield | Purity | Impurity **VIII** | Impurity **IX** |
|---|---|---|---|---|---|
| 1.0 mL/g | 1 : 5 | 83% | 98.55% | 0.78% | 0.27% |
| 2.0 mL/g | 1.33 : 1 | 82% | 99.22% | 0.50% | 0.10% |
| 3.0 mL/g | 1 : 2 | 80% | 99.45% | 0.20% | 0.09% |
| 4.0 mL/g | 1:2 | 74% | 99.41% | 0.26% | 0.07% |

The compound of the present invention is purified by the present method. When ethyl acetate or dichloromethane is used as an organic solvent, a compound I with high purity can be obtained by using less organic solvent and water, and less impurity **VIII** and impurity **IX** are contained in the product. If the contents of the two impurities are higher, it is difficult to obtain an acceptable finished active pharmaceutical ingredient product via subsequent purification, and the introduction of a relatively large amount of these two impurities into the drug may cause unpredictable safety issues.

### Comparative examples:

The purification method of Example 3 was followed, except that water - ethyl acetate was replaced with a solvent system given in Table 4, and different solvent systems and mixing ratios were used, respectively, so as to refine the crude compound **I** obtained in Example 2. The yields, purity, and main impurities were compared, and the results were shown in Table 4.

**Table 4 Results of refining using different solvent systems and mixing ratios**

| Solvent for refining | Amount of organic solvent | Organic solvent : Water (weight ratio) | Yield | Purity | Impurity **VIII** | Impurity **IX** |
|---|---|---|---|---|---|---|
| Isopropyl acetate - water | 5.0 mL/g | 1 : 2 | 78% | 98.23% | 0.84% | 0.19% |
| | 9.0 mL/g | 1 : 1.5 | 74% | 98.85% | 0.56% | 0.12% |
| Ethanol | 5.0 mL/g | - | 65% | 99.10% | 0.36% | 0.09% |
| Toluene - ethanol = 1 : 1 | 5.0 mL/g | - | 68% | 99.21% | 0.39% | 0.13% |
| Toluene - acetonitrile = 1:1 | 5.0 mL/g | - | 85% | 94.10% | 3.36% | 0.30% |

When isopropyl acetate - water is used as the solvent for the purification of compound I, and the volume-to-weight ratio of isopropyl acetate to the compound is 5.0 mL/g, the compound cannot be completely dissolved, and a clear solution can be obtained only when the amount of isopropyl acetate reaches 9.0 mL/g. When an organic solvent such as toluene, acetonitrile, ethanol, or a mixed solvent thereof is used as the solvent for the purification of the compound of the present invention, a higher amount of the organic solvent is likewise required, which leads to significant solvent recovery issues or insufficient refining effects.

It should be understood that the above detailed description and accompanying examples are only exemplary and are not considered to limit the scope of the present invention, which is limited only by the appended claims and equivalents thereof. Various changes and modifications to the disclosed examples will be apparent to those skilled in the art. Such changes and modifications, including but not limited to those related to the method of the present invention, or any combination thereof, may be implemented without departing from the spirit and scope of the present invention.

## Claims

1. A method for preparing a compound represented by formula II, **characterized by** 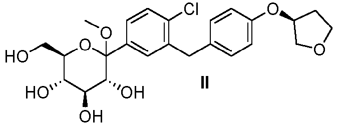 comprising the following steps:
(a) in an organic solvent, reacting a compound represented by formula III 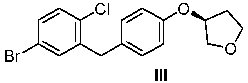 with a compound represented by formula IV under the action of organic metal lithium 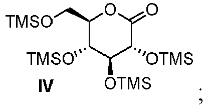
(b) quenching the reaction of step (a) with water to obtain a mixed solution;
(c) subjecting the mixed solution obtained in step (b) to phase separation into an organic phase and an aqueous phase, and concentrating the organic phase to obtain a compound represented by formula V 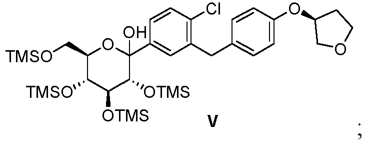 and
(d) reacting the compound represented by formula V obtained in step (c) with methanol under an acidic condition to obtain the compound represented by formula II.

2. The method according to claim 1, **characterized in that** the step (b) is carried out in such a manner that the reaction solution of step (a) is slowly added to water, with the temperature of quenching being controlled at 0-10°C.

3. The method according to claim 2, **characterized in that** the water is pre-cooled to 0-5°C.

4. The method according to claim 1, **characterized in that** the phase separation in step (c) is carried out in such a manner that the mixed solution obtained in step (b) is heated to 20-30°C with stirring, so as to separate the organic phase from the aqueous phase.

5. The method according to claim 1, **characterized in that** the reaction in step (a) is carried out at a temperature of -90°C to -60°C, preferably -80°C to - 70°C; and the reaction in step (d) is carried out at a temperature of -80°C to 40°C.

6. A method for preparing a compound represented by formula I, **characterized by** comprising the following step: 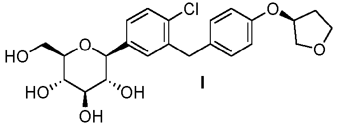 (e) reacting the compound represented by formula II prepared by the method according to any one of claims 1-5 with a reducing reagent in the presence of a Lewis acid in a solvent to obtain the compound represented by formula I.

7. A method for purifying the compound represented by formula I, **characterized by** comprising the following steps: 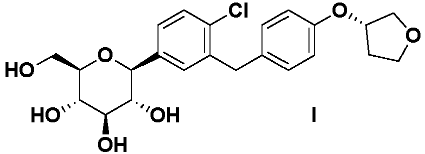
(e) reacting the compound represented by formula II prepared by the method according to any one of claims 1-5 with a reducing reagent in the presence of a Lewis acid in a solvent to obtain a compound represented by formula I, and removing the solvent to obtain a crude product of the compound of formula I;
(f) adding the crude product of the compound represented by formula I obtained in step (e) to a mixed solvent of an organic solvent and water, and heating same with stirring to form a mixed solution;
(g) optionally, cooling the mixed solution obtained in step (f) to crystallise the compound; and
(h) filtering same to obtain the crystals of the compound represented by formula I; wherein
the organic solvent is selected from ethyl acetate and dichloromethane,
wherein the weight ratio of ethyl acetate to water is in a range of 1 : 10 to 5 : 1, and
wherein the weight ratio of dichloromethane to water is in a range of 1 : 10 to 10 : 1.

8. The method according to claim 7, **characterized in that** the volume-to-weight ratio of ethyl acetate to the compound is 1.0 mL/g - 3.0 mL/g, preferably 1.5 mL/g - 2.0 ml/L.

9. The method according to claim 7, **characterized in that** the volume-to-weight ratio of dichloromethane to the compound is 1.5 mL/g - 4.0 ml/L, preferably 2.0 mL/g - 3.0 ml/L.

10. The method according to claim 7, **characterized in that** the weight ratio of ethyl acetate to water is in a range of 1 : 8 to 1 : 1, further preferably in a range of 1 : 6 to 1 : 2, still further preferably in a range of 1 : 2 to 1 : 3.3.

11. The method according to claim 7, **characterized in that** the weight ratio of dichloromethane to water is in a range of 1 : 5 to 5 : 1, further preferably in a range of 1 : 3 to 4 : 1, still further preferably in a range of 1 : 3.3 to 1 : 2.

12. The method according to claim 7, **characterized in that** in step (f), when the organic solvent is ethyl acetate, the temperature of heating with stirring is 55-65°C, and when the organic solvent is dichloromethane, the temperature of heating with stirring is 25-45°C.

13. The method according to claim 7, **characterized in that** the cooling in step (g) is carried out in such a manner that the temperature is gradually reduced to 0-5°C.
